# EUROPEAN PATENT APPLICATION

(11) **EP 1 421 934 A1**
(43) Date of publication of application: **26.05.2004**
(21) Application number: 02755820.4
(22) Date of filing: 05.08.2002
(51) Int. Cl.: A61K 31/166, A61P 19/02, A61P 43/00

(54) **REMEDIAL AGENT FOR ARTHROSIS DEFORMANS**

(30) Priority: 06.08.2001 JP 2001237345
(71) Applicant: ONO PHARMACEUTICAL CO., LTD., Osaka-shi, Osaka 541-8526 (JP)
(72) Inventor: MAEDA, Yoshizo, Mishima-gun, Osaka 618-8585 (JP); YAMASHITA, Kazunori, Mishima-gun, Osaka 618-8585 (JP); OGAWA, Koji, Mishima-gun, Osaka 618-8585 (JP)
(74) Representative: Henkel, Feiler & Hänzel
(86) International application number: PCT/JP2002/007961
(87) International publication number: WO 2003/013494

(57) **Abstract**

A therapeutic and/or prophylactic drug for osteoarthritis, comprising a derivative of hydroxamic acid, which is represented by the formula (I) 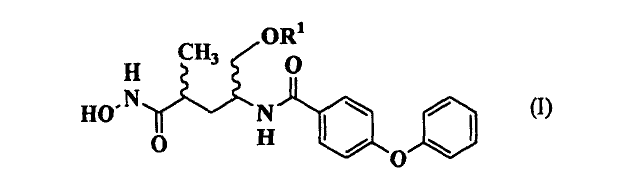 (wherein R¹ represents a halogen atom, C1-8 alkyl, or C1-8 alkyl substituted with -OR², in which R² represents a halogen atom, C1-8 alkyl, benzyl, or C1-8 alkyl substituted with C1-8 alkoxy), or the non-toxic salts with inhibitory effect of matrix metalloproteinase.

## Description

### TECHNICAL FIELD

The present invention relates to a medicine for osteoarthritis.

More particularly, the present invention relates to therapeutic and/or prophylactic drugs for osteoarthritis, comprising derivatives of hydroxamic acid, which are represented by the formula (I) 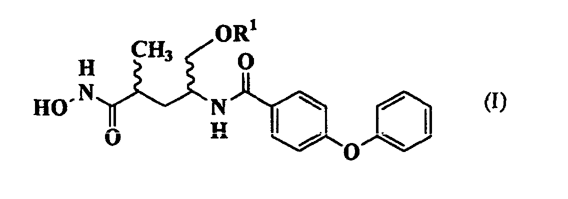 (all signs in the formula represent the same meanings as postscripts.) having inhibitory activities for matrix metalloproteinases or these non-toxic salts, as active ingredients.

### BACKGROUND OF THE INVENTION

Matrix metalloproteinases (hereafter, it is abbreviated with MMP.) are neutral metalloproteinases that have zinc (hereafter, it is abbreviated with Zn²⁺) in the active center. So far, at least, the molecular species more than 20 species with different primary structure have been identified. Concretely, interstitial collagenase (MMP-1), neutrophil collagenase (MMP-8), collagenase-3 (MMP-13), gelatinase A (MMP-2), gelatinase B (MMP-9), stromelysin-1 (MMP-3), stromelysin-2 (MMP-10), matrilysin (MMP-7), metalloelastase (MMP-12), etc. are enumerated.

They are effective in growth and tissue remodeling of articulation tissue, bone tissue and connective tissue by degrading collagen, laminin, proteoglycans, fibronectin, elastin, gelatin, etc. under physiological condition. However, it is considered that disorganization of various tissues under morbid condition was caused by increase in expressions or activities of MMPs, which was caused by bankruptcy of MMP modulation. For example, it has been reported that MMP-1, 2, 3, 8, 9, and 13 could be highly expressed in a part of damaged cartilage of patients with osteoarthritis, and deeply concerned with degradation of cartilage [Igaku no ayumi, 182, 549-553 (1997); J. Clin. Invest., 84, 678-685 (1989)].

On the other hand, osteoarthritis (OA) is the majority of arthralgia for middle-aged and elderly patients. A decrease in resilienc, abrasion, and fissure, etc. was caused by retrograde degeneration of articular cartilage, and so increasing chondral ossification and osteoplasty around joint was encouraged, at last, an articular cartilage was disappeared and so a bone was outcropped at joint, and arthralgia and pooling of synovial fluid were caused. A favorite site was knee, crotch, spinal cord, cubitus, shoulder or finger. A lot of the causes are uncertain.

Non-steroidal analgesic and anti-inflammatory drugs were used as drugs of first alternative for the above disease. Besides, intraarticular injection of hyaluronic acid preparation, which improves lubrication function of joint and has anti-inflammatory effect, was carried out. However, These are only symptomatic therapy, and a fundamental method of the treatment has not been established.

For example, specifications of GB2268934 and EP606046 are enumerated as patent applications that enumerate osteoarthrosis as subject diseases of compound with MMP inhibitory effect. It has been reported that CGS-27023A: 2(R)-[N-(4-methoxyphenylsulfonyl)-N-(3-pyridylmethyl)amino]-3-methylbutanohydroxamic acid hydrochloride, which was a MMP inhibitor, could be effect on a model of OA in rabbits [Inflamm Res 44, Supplemet 2, S117-118 (1995)]. However, there are no MMP inhibitors as therapeutic and/or prophylactic agents for OA, and precious few compound with MMP inhibitory activity under development for OA.

### DISCLOSURE OF THE INVENTION

As previously described, it was eagerly looking forward to supply a therapeutic and/or prophylactic agents for OA, and expected that MMP inhibitors will become the therapeutic and/or prophylactic agents. However, it is already natural that all known MMP inhibitory agents are not necessarily effective in OA, and it is not achieved still.

Under the above condition, as a result that the present inventors examined assiduously, they have first found that derivatives of hydroxamic acid, which are represented by the formula (I), or the non-toxic salts could be useful for OA, and accomplished the present invention.

The present invention relates to therapeutic and/or prophylactic drugs for osteoarthritis comprising derivatives of hydroxamic acid, which are represented by the formula (I) 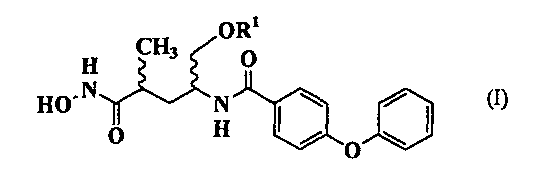 (wherein R¹ represents a halogen atom, C1-8 alkyl, or C1-8 alkyl substituted with -OR², in which R² represents a halogen atom, C1-8 alkyl, benzyl, or C1-8 alkyl substituted with C1-8 alkoxy(s), except for N-hydroxy-5-ethoxymethyloxy-2(S)-methyl-4(S)-(4-phenoxybenzoyl)aminopentanamide.) or the non-toxic salts.

The derivatives of hydroxamic acid, which are represented by the formula (I), has been described in a specification of WO99/19296 as compounds with MMP inhibitory activity.

In the above specification, it have been described that the derivatives of hydroxamic acid including the compounds represented by the formula (I), have MMP inhibitory activity, so they are useful for treatment and/or prevention of diseases, for example, rheumatoid diseases, arthrosteitis, unusual bone resorption, osteoporosis, periodontitis, interstitial nephritis, arteriosclerosis, pulmonary emphysema, cirrhosis, cornea injury, cornea ulcer, metastasis, invasion or growth of tumor cells, autoimmune disease (Crohn's disease, Sjogren's syndrome), disease caused by vascular emigration or infiltration of leukocytes, arterialization, multiple sclerosis, arota aneurysm, endometriosis, etc. However, there is no description that the compounds represented by the formula (I) are useful for OA.

Although some relations between MMP and OA were known, the present inventors applied some MMP inhibitors to a partial meniscectomy model of osteoarthritis rabbits, then only compounds represented by the formula (I) showed efficiency.

### DETAILED DESCRIPTION OF THE INVENTION

C1-8 alkyl in the formula (I) of the present invention represents methyl, ethyl, propyl, butyl, pentyl, hexyl, heptyl, octyl group, or these isomers.

In the formula (I), C1-8 alkyl substituted with -OR² represents methyl, ethyl, propyl, butyl, pentyl, hexyl, heptyl, or octyl group, which is substituted with a single -OR², or these isomers.

In the formula (I), C1-8 alkyl substituted with C1-8 alkoxy(s) represents methoxy, ethoxy, propoxy, butoxy, pentyloxy, hexyloxy, heptyloxy, or octyloxy group; methyl, ethyl, propyl, butyl, pentyl, hexyl, heptyl, octyl group, or these isomers, which is substituted with a group selected from these isomers.

In the formula (I), 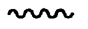 sign represents a bond that shows an isomer with asymmetric carbon or the mixture, concretely, 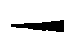 sign represents a bind forward space, and 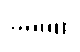 sign represents a bind backward space, or these mixture.

In the present invention, isomers contain all isomers as long as it does not especially direct it. For example, straight chains and branched chains are contained in alkyl and alkoxy group. In addition, isomers with asymmetric carbons, etc. (R, S body, α, β body, enantiomer, and diastereomer), optical isomers with optical rotation (D, L, d, and I body), polarity bodies separated by chromatograph (high polarity body and low polarity body), balanced compounds, these arbitrary ratio compounds, and racemic mixtures are contained in the present invention.

All groups represented by R¹ are suitable for R¹ in the formula (I), more suitably, a hydrogen atom, C1-4 alkyl, or C1-4 alkyl substituted with a single -OR².

All groups represented by R² are suitable for R² in the formula (I), more suitably, a hydrogen atom, C1-4 alkyl, benzyl, or C1-4 alkyl substituted with a single C1-4 alkoxy.

Especially, as R¹ in this specification, a hydrogen atom and methyl, ethyl, methoxymethyl, ethoxymethyl, ethoxyethyl, benzyloxymethyl, methoxymethoxymethyl, or methoxyethoxymethyl group are suitable.

As concrete compounds used for the present invention,
N-hydroxy-5-hydroxy-2(S)-methyl-4(S)-(4-phenoxybenzoyl)aminopentanamide,
N-hydroxy-5-methoxymethyloxy-2(S)-methyl-4(S)-(4-phenoxybenzoyl)aminopentanamide,
N-hydroxy-5-ethoxymethyloxy-2(R)-methyl-4(R)-(4-phenoxybenzoyl)aminopentanamide,
N-hydroxy-5-ethoxymethyloxy-2(R)-methyl-4(S)-(4-phenoxybenzoyl)aminopentanamide,
N-hydroxy-5-benzyloxymethyloxy-2(S)-methyl-4(S)-(4-phenoxybenzoyl)aminopentanamide, or
N-hydroxy-5-(2-methoxyethoxy)methyloxy-2(S)-methyl-4(S)-(4-phenoxybenzoyl)aminopentanamide, or these non-toxicity salts are enumerated.

The compounds used in the present invention may be used as the following, non-toxic salts, which non-toxic and water-soluble one are suitable.

As suitable salts, alkaline earth salts (potassium, sodium, etc.), alkaline earth metal salts (calcium, magnesium, etc.), ammonium salts, pharmaceutically acceptable organic amine salts (tetramethylammonium, triethylamine, methylamine, dimethylamine, cyclopentylamine, benzylamine, phenethylamine, piperidine, monoethanolamine, diethanolamine, tris(hydroxymethyl)amine, lysine, arginine, N-methyl-D-glucamine, etc.) are enumerated. The compounds represented by formula (I) and their salts may be converted into the hydrate by a conventional manner.

The compounds represented by the formula (I) can be manufactured by the method described in a specification of WO99/19296.

### BRIEF DESCRIPTION OF DRAWINGS

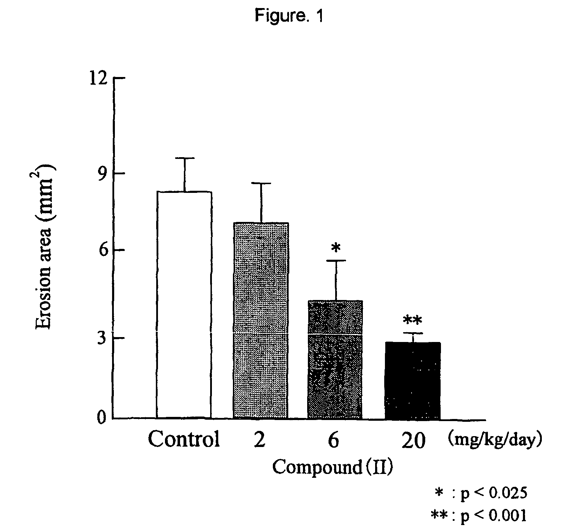

Figure 1 shows inhibitory effect on erosion area of thigh bone, when various doses of N-hydroxy-5-hydroxy-2(S)-methyl-4(S)-(4-phenoxybenzoyl) aminopentanamide (compound (II)) twice a day were administered.

### BEST FORM TO EXECUTE INVENTION

Efficiencies of the compounds in the present invention on osteoarthrosis were proven, for example, by an experiment of a partial meniscectomy model in rabbits.

### Experimental method:

Female rabbits (Kbs: NZW (Healthy)) were preliminarily bred for a week and subjected to ablative operation of meniscus by the following method.

Each animal was anesthetized by a subcutaneous injection of 2% parenteral celactal (0.05 ml/kg) to the poll and an intravenous injection of nembutal (20 mg/kg) to the marginal auricle. Their right knees were disinfected with Iodine tincture that dilutes five times with distilled water. If necessary, local anesthesia was performed by dropping 2% of parenteral xylocaine to incised part.

Then, skins and articular capsules outside knees of right hind legs were incised at right angle to patellar ligament, fibular collateral ligaments and sesamoid ligaments were transected. In that case, parenteral solution of bosmin was dropped for the arrest of hemorrhage. Tissues connecting with tissues forward lateral meniscuses were picked up with forceps, the meniscuses were drawn out forward, and 1/3 of the centra were excised. After washing the operated area with saline solution, the synoviums and articular capsules were sutured together. Then muscular coats and skins were sutured, respectively.

After operation, potassium penicillin G (5000 U/animal) and streptomycin sulfate (100 mg/animal) were intramuscularly administered to left hind leg to prevent infection.

While they are bred until being dissected on 7 days after operative, as a subject compound, N-hydroxy-5-hydroxy-2(S)-methyl-4(S)-(4-phenoxybenzoyl)aminopentanamide (hereafter, compound (II)) represented by the following (II) 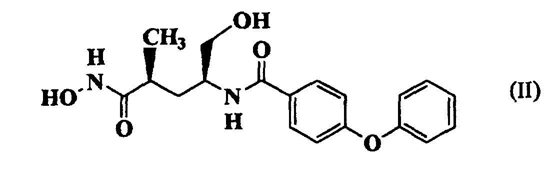 , which the applied dose per a day are 1, 3, and 10 mg/kg, respectively, were administered twice a day.

After anesthesia by an intravenous injection of parenteral nembutal (40 mg/kg) to marginal auricle, the animals were made to die by venesection and gathered their right knees of joint part. The knee joints were incised, and the thigh bones and the shinbone heads were gathered. They were stored in 10% of formalin neutral buffer at room temperature. After collecting all samples and masking them, their erosion areas were measured with a stereoscopic microscope.

Their erosive areas of subject compound groups were compared to one of a vehicle group by using statistical analysis of Williams multiple comparison test. The result was shown in Figure 1.

The above experimental model, which has been generally acknowledged as osteoarthritis model, can induce the cartilage destruction that closely resembles to human osteoarthrosis. It was understood that the compound represented by the formula (I) could be highly effective in this model. Therefore, it was considered that the compounds represented by the formula (I) could be effective in osteoarthrosis.

### Toxicity:

It was considered that the toxicities of the compounds represented by the formula (I) could be very low and safe to use as a medicine. For example, the lowest lethal dose in oral administration of compound (II) to rat once was 2000 mg/kg or more.

### Application for pharmaceuticals:

The compounds of the present invention, which are hydroxamic acid derivatives represented by the formula (I) or the non-toxic salts with inhibitory activities of matrix metalloproteinases, for example, gelatinase, stromelysin, or collagenase, etc., are useful for the treatment and/or prevention of osteoarthritis in animals including human, especially human.

The compounds represented by the formula (I) or the non-toxicity salts,
1) to supplement and/or reinforce the prophylactic and /or therapeutic effect of the compounds,
2) to improve the movement and absorption of the compounds, and to decrease the dosage, and/or
3) to reduce the side effects of the compounds, may be administered as a other concomitant drug combining with other medicines.

Other concomitant drugs with the compounds represented by the formula (I) and other medicines may be administered in the combined form that mixes both elements in the formulation or in the form administered as separate formulation. In the form administered as separate formulation, the different and simultaneous administration are included. In the different administration, the compounds represented by the formula (I) may be previously administered, followed by other medicines, or other medicines may be previously administered, followed by the compounds represented by the formula (I). Each medication method may be different or same.

Illnesses that the prophylactic and/or therapeutic effects of the above other concomitant drugs can be expected, which only has to be one that the prophylactic and/or therapeutic effects of the compounds represented by the formula (I) is/are supplemented and/or reinforced, are not limited especially.

As other medicines to supplement and/or reinforce the prophylactic and/or therapeutic effects of the compounds represented by the formula (I) on osteoarthrosis, for example, non-steroidal anti-inflammatory drug, steroid drug, immunosuppressive drug, antiflammatory exogenous enzyme, cartilage protective drug, T cell inhibitor, TNFα inhibitor, prostaglandin synthase inhibitor, IL-6 inhibitor, interferon γ agonist, IL-1 inhibitor, prostaglandins, and phosphodiesterase 4 inhibitor, etc. are enumerated.

As non-steroidal anti-inflammatory drugs, for example, sasapyrine, sodiumsalicylate, aspirin, aspirin, dialuminates combination, diflunisal, indomethacin, suprofen, ufenamate, dimethyl isopropyl azulene, bufexamac, felbinac, diclofenac, tolmetin sodium, clinoril, fenbufen, puroglumetacine, indomethacin famesyl, acemetacin, proglumetacin maleate, amfenac sodium, mofezolac, etodolac, ibuprofen, ibuprofen piconol, naproxen, flurbiprofen, flurbiprofen axetil, ketoprofen, fenoprofen calcium, tiaprofen, oxaprozin, pranoprofen, loxoprofen sodium, aluminoprophen, zaltoprofen, mefenamic acid, mefenamic acid aluminum, tolfenamic acid, floctafenine, ketophenylbutazone, oxyphenbutazone, piroxicam, tenoxicam, ampiroxicam, napageln ointment, epirizole, tiaramide hydrochloride, tinoridine hydrochloride, emorfazone sulpyrine, migrenin, saridon, sedes-g, amipylo-n, mysis relicta, pillow, sorbon, pyrine drug for common cold, acetaminophen, phenacetin, dimetotiazine mesilate, simetride combination drug, and non-pyrine drug for common cold, etc. are enumerated.

In steroid drugs, for example, as drugs for external use, clobetasol propionate, diflorazone diacetate, fluocinonide, mometasone furate, betamethasone dipropionate, betamethasone butyrate propionate, betamethasone valerate, difluprednate, budesonid, diflucortolone valerate, amcinonide, halcinonide, dexamethasone, dexamethasone propionate, dexamethason valerate, dexamethasone acetate, hydrocortisone acetate, butyric acid hydrocortisone, hydrocortisone butyrate propionate, deprodone propionate, prednisolone valerate-acetate, fluocinolone acetonide, beclometasone dipropionate, triamcinolone acetonide, flumetasone pivalate, alclometasone propionate, clobetasone butyrate, prednisolone, beclomethasone propionate, and fludroxycortide, etc. are enumerated.

As internal medicines and injections, cortisone acetate, hydrocortisone, hydrocortisone sodium phosphate, hydrocortisone sodium succinate, fludrocortisone acetate, Prednisolone, prednisolone acetate, prednisolone sodium succinate, prednisolone butylacetate, prednisolone sodium phosphate, halopredone acetate, methylprednisolone, methylprednisolone acetate, sodium methylprednisolone succinate, triamcinolone, triamcinolone diacetate, triamcinolone acetonide, dexamethasone, dexamethasone acetate, dexamethasone sodium phosphate, dexamethasone palmitate, paramethasone acetate, and betamethasone, etc. are enumerated.

As inhalants, beclometasone dipropionate, fluticasone propionate, budesonide, flunisolide, triamcinolone, ST-126P, ciclesonide, dexamethasone palmitate, mometasone furoate, prasterone sulfonate, deflazacort, methylprednisolone suleptanate, and methylprednisolone sodium succinate, etc. are enumerated.

As cartilage protective drugs, for example, sodium hyaluronate, glucosamine, chondroitin sulfuric acid, and polysulfate glycosaminoglycan, etc. are enumerated.

Mass ratio of the compounds represented by the formula (I) to other medicines is not especially limited.

Other medicines may be administered combining with two arbitrary kinds or more.

And, in other medicines that supplement and/or reinforce prophylactic and/or therapeutic effects of the compounds represented by the formula (I), not only one that has been found by present based on the above mechanism but also one that will be found in the future are contained.

To use the compounds represented by the formula (I) or other concomitant drugs containing the compounds represented by the formula (I) and other medicines by the above purpose, they are usually administered systemically or locally, and orally or parenterally.

Applied doses are determined depending upon, for example, age, body weight, symptom, the desired therapeutic effect, the route of administration, and the duration of the treatment. Generally, a dose from 1 ng to 100 mg per an adult is orally administrated from once to several times per day, parenterally from 0.1 ng to 100 mg, or intravenously from 1 to 24 hours per day, continuously.

As above mentioned, because the dose depends upon various conditions, there are cases in which the dose is lower or higher than the above dose.

When the compounds represented by the formula (I) or other concomitant drugs containing them and other medicines are administered, they are used as solid medicines and liquid medicines for internal use, and injections, liquid medicines, external preparations, suppositoriums, eye drops, and inhalants, etc. for parenteral administration.

Solid forms for oral administration may include compressed tablets, pills, capsules, dispersible powders, and granules, etc. Capsules may include hard capsules and soft capsules.

In such solid forms for oral administration, one or more of active compound(s) may be admixed itself (themselves) or with vehicles (lactose, mannitol, glucose, microcrystalline cellulose, starch, etc.), binders (hydroxypropyl cellulose, polyvinylpyrrolidone or magnesium metasilicate aluminate, etc.), disintegrants (cellulose calcium glycolate, etc.), lubricants (magnesium stearate, etc.), stabilizers, and/or solubilizers (glutamic acid or aspartic acid, etc.) and used manufacturing pharmaceutically according to well known methods. If desired, they may be coated with coating agents (sugar, gelatin, hydroxypropyl cellulose or hydroxypropylmethyl cellulose phthalate, etc.) or coated with two or more films. Further, they may include capsules comprising absorbable materials such as gelatin.

Liquid forms for oral administration may include pharmaceutically acceptable solutions, suspensions, emulsions, syrups and elixirs, etc. In such forms, one or more of active compound(s) may be dissolved, suspended or emulized into diluent(s) (such as purified water, ethanol or a mixture thereof) generally used. Besides this liquid forms may also comprise wetting agent, suspending agent, emulsifying agent, sweetening agent, flavoring agent, aroma, preservatives, or buffer, etc.

In external preparations for parenteral administration, for example, ointment, gel, cream, fomentation, patch, liniment, aerosol, inhalant, spray, aerosol, and nasal drop, etc. are contained. They contain one or any more activators, and are prepared by a well-known method or usually used prescription.

Ointments are manufactured by a well-known or usually used prescription. For example, they are made by incorporating or melting one or more activators to the base. Ointment bases are chosen from the well-known or usually used one. They are used mixing a single or two kinds or more chosen from, for example, higher fatty acid or higher fatty acid ester (adipic acid, myristic acid, palmitic acid, stearic acid, oleic acid, adipic acid ester, myristic acid ester, retinol palmitate, stearic acid ester, and oleic acid ester, etc.), wax (wax, spermaceti, and ceresin, etc.), surfactant (polyoxyethylene alkyl ether phosphate ester, etc.), higher alcohol (cetanol, stearyl alcohol, and cetostearyl alcohol, etc.), silicone oil (dimethylpolysiloxane), hydrocarbon (hydrophilic petrolatum, white petrolatum, purified lanolin, and liquid paraffin, etc.), glycol (ethylene glycol, diethylene glycol, propylene glycol, polyethylene glycol, and macrogol, etc.), vegetable oil (castor oil, olive oil, sesame oil, and turpentine oil, etc.), animal oil (mink oil, yolk oil, squalane, and squalene, etc.), water, absorption promoter, and rash inhibitor. In addition, they may contain moisturizing agent, preservative, stabilizer, antioxidant, and flavor, etc.

Gels are manufactured by a well-known or usually used prescription. For example, they are manufactured by melting one or more activators to base. Gel bases are chosen from the well-known one or the one usually used one. They are used mixing single or two kinds or more chosen from, for example, lower alcohol (ethanol and isopropyl alcohol, etc.), gelatinizer (carboxymethylcellulose, hydroxyethyl cellulose, hydroxypropylcellulose, and ethyl cellulose, etc.), neutralizer (triethanolamine and diisopropanolamine, etc.), surfactant (polyethyleneglycol monostearate, etc.), gums, water, absorption promoter, and rash inhibitor. In addition, they may contain preservative, antioxidant, and flavor, etc.

Creams are manufactured by a well-known or usually used prescription. For example, they are manufactured by melting one or more activators to base and emulsifying. Creams are chosen from the well-known or usually used one. They are used mixing a single or two kinds or more chosen from, for example, higher fatty acid ester, lower alcohol, hydrocarbons, polyhydric alcohol (propylene glycol, 1, 3-butylene glycol, etc.), higher alcohol (2-hexyldecanol and cetanol, etc.), emulsifier (polyoxyethylene alkyl ether and fatty acid ester, etc.), water, absorption promoter, and rash inhibitor. In addition, they may contain preservative, antioxidant, and flavor, etc.

Fomentations are manufactured by a well-known or usually used prescription. For example, they are manufactured by melting one or more activators to base, kneading and roll spreading on support medium. Compress bases are chosen from the well-known or usually used one. They are used mixing a single or two kinds or more chosen from, for example, thickener (polyacrylic acid, polyvinylpyrrolidone, acacia, starch, gelatin, and methylcellulose, etc.), humectants (urea, glycerin, and propylene glycol, etc.), filler (china clay, zinc oxide, talc, calcium, and magnesium, etc.), water, solubilizer, tackifier, and rash inhibitor. In addition, they may contain preservative, antioxidant, and flavor, etc.

Patchs are manufactured by a well-known or usually used prescription. For example, they are manufactured by melting one or more activators to base, kneading and roll spreading on support medium. Bases for patch are chosen from well-known or usually used one. For example, one or two kinds or more chosen from high molecular group base, fats and oils, higher fatty acid, tackifier, and rash inhibitor is/are used by mixing. In addition, they may contain preservative, antioxidant, and flavor, etc.

Liniments are manufactured by a well-known or usually used prescription. For example, one or more activators is/are manufactured by being dissolved, suspended or emulsified to one or two kinds or more chosen from water, alcohol (ethanol and polyethyleneglycol, etc.), higher fatty acid, glycerin, soap, or emulsifier, etc. In addition, they may contain preservative, antioxidant, and flavor, etc.

Aerosols, inhalants, and aerosols may include stabilizers such as sodium hydrogen sulfite besides diluent usually used and isotonicity medicine like buffer giving isotonicity, for example, sodium chloride, sodium citrate, or citrate. The manufacturing method of aerosol has been described in detail, for example, in U.S. patent No.2,868,691 and No.3,095,355.

Injections for parenteral administration may include solution, suspension, emulsion, and solid injections which are used dissolving or suspending in solvent for time of use. The injections are used dissolving, suspending, and emulsifying one or more of active compound(s) to solvent. As solvent, distilled water for injection, physiological salt solution, vegetable oil, propylene glycol, polyethylene glycol, alcohols like ethanol, or their mixtures may be used. Further, these injections may contain stabilizer, solubilizer (glutamic acid, aspartic acid or POLYSORBATE80 (registered trade mark), etc.), suspending agent, emulsifying agent, soothing agent, buffer, preservative, etc. These may be sterilized at a final step or prepared by aseptic manipulation. These may also be used by being manufactured as sterile solid forms, for example, freeze-dried products and dissolved in aseptic or sterile water or other sterile diluent(s) for injection immediately before using.

Inhalants for parenteral administration may include aerosol agent, inhalant powder or inhalant liquid, which may be used by being dissolved or suspended in water or other suitable media before using.

Inhalants are manufactured based on a well-known method.

For example, inhalant liquids are prepared properly selecting, if necessary, preservative (benzalkonium chloride and paraben, etc.), coloring agent, buffer (sodium phosphate and sodium acetate, etc.), tonicity agent (sodium chloride and concentrated glycerin, etc.), thickener (carboxyvinyl polymer, etc.), and absorption enhancers, etc.

Inhalant powders are preparated properly selecting, if necessary, lubricant (stearic acid and the salt, etc.), binder (starch and dextrin, etc.), filler (lactose and cellulose, etc.), coloring agent, preservative (benzalkonium chloride and paraben, etc.), and absorption enhancer, etc.

When inhalant liquids are administered, sprayer (atomizer and nebulizer) is usually used, and when inhalant powders are administered, an inhalation administering machine for powder is usually used.

Pessaries, etc for a suppository and intravaginal administration to intrarectally administrate with common procedure, which contains one or more activators are included as other compositions for parenteral administration.

### Formulation example 1:

The following components were admixed in conventional method and punched out to obtain 100 tablets each containing 50 mg of active ingredient in a tablet.
- N-hydroxy-5-hydroxy-2(S)-methyl-4(S)-(4-phenoxybenzoyl)aminopentanamide 5.0 g
- Carboxymethylcellulose calcium (disintegrator) 0.2 g
- Magnesium stearate (lubricant) 0.1 g
- Crystallite cellulose 4.7 g

### Formulation example 2:

After the following components were admixed in conventional method, the solution was sterilized in conventional manner, placed 5 ml portions into ampoules and freeze-dried to obtain 100 ampoules each containing 20 mg of the active ingredient.
- N-hydroxy-5-hydroxy-2(S)-methyl-4(S)-(4-phenoxybenzoyl)aminopentanamide 2.0 g
- mannitol 20 g
- distilled water 500 ml

## Claims

1. A therapeutic and/or prophylactic drug for osteoarthritis, comprising a derivative compound of hydroxamic acid, which is represented by the formula (I), 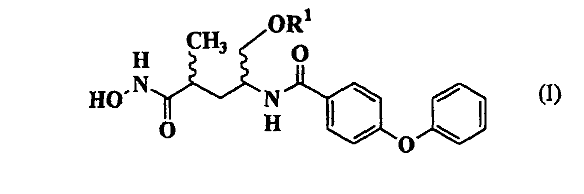 wherein R¹ represents a halogen atom, C1-8 alkyl, or C1-8 alkyl substituted with -OR², in which R² represents a halogen atom, C1-8 alkyl, benzyl, or C1-8 alkyl substituted with C1-8 alkoxy(s), except for N-hydroxy-5-ethoxymethyloxy-2(S)-methyl-4(S)-(4-phenoxylbenzoyl)pentanamide, or a non-toxic salt thereof.

2. The therapeutic and/or prophylactic drug for osteoarthritis, comprising the derivative compound of hydroxamic acid or the non-toxic salt of claim 1, wherein R¹ is a halogen atom, C 1-4 alkyl, or C1-4 alkyl substituted with a single -OR², in which R² is a halogen atom, C1-4 alkyl, benzyl, or C1-4 alkyl substituted with C1-4 alkoxy(s).

3. The therapeutic and/or prophylactic drug for osteoarthritis, comprising the derivative compound of hydroxamic acid or the non-toxic salt of claim 2, wherein R¹ is a hydrogen atom, methyl, ethyl, methoxymethyl, ethoxymethyl, ethoxyethyl, benzyloxymethyl, methoxymethoxymethyl, or methoxyethoxymethyl.

4. The therapeutic and/or prophylactic drug for osteoarthritis, comprising the compound of claim 1, wherein the compound is
(1) N-hydroxy-5-hydroxy-2(S)-methyl-4(S)-(4-phenoxybenzoyl)aminopentanamide,
(2) N-hydroxy-5-methoxymethyloxy-2(S)-methyl-4(S)-(4-phenoxybenzoyl)aminopentanamide,
(3) N-hydroxy-5-ethoxymethyloxy-2(R)-methyl-4(R)-(4-phenoxybenzoyl)aminopentanamide,
(4) N-hydroxy-5-ethoxymethyloxy-2(R)-methyl-4(S)-(4-phenoxybenzoyl)aminopentanamide,
(5) N-hydroxy-5-benzyloxymethyloxy-2(S)-methyl-4(S)-(4-phenoxybenzoyl)aminopentanamide,
(6) N-hydroxy-5-(2-methoxyethoxy)methyloxy-2(S)-methyl-4(S)-(4-phenoxybenzoyl)aminopentanamide, or a non-toxicity salt thereof.
